(19) Europäisches Patentamt
European Patent Office
Office européen des brevets

(11) **EP 3 967 370 A1**

(12) **EUROPEAN PATENT APPLICATION**
published in accordance with Art. 153(4) EPC

(43) Date of publication:
**16.03.2022 Bulletin 2022/11**

(21) Application number: **20805037.7**

(22) Date of filing: **08.04.2020**

(51) International Patent Classification (IPC):
**A61P 43/00** (2006.01)  **A61P 17/00** (2006.01)
**A61Q 19/00** (2006.01)  **A61K 36/062** (2006.01)
**A61K 36/899** (2006.01)  **A61K 8/9728** (2017.01)
**A61K 8/9794** (2017.01)

(52) Cooperative Patent Classification (CPC):
**A61K 8/9728; A61K 8/9794; A61K 36/062;
A61K 36/899; A61P 17/00; A61P 43/00;
A61Q 19/00**

(86) International application number:
**PCT/JP2020/015809**

(87) International publication number:
**WO 2020/230483 (19.11.2020 Gazette 2020/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.05.2019 JP 2019089438**

(71) Applicant: **Kohsei Industrial Co., Ltd.
Ibi-gun, Gifu 501-0535 (JP)**

(72) Inventors:
• **NAKAMURA Masahiko
Ibi-gun Gifu 501-0535 (JP)**
• **MIYAI Daisuke
Ibi-gun Gifu 501-0535 (JP)**
• **KURIMOTO Shouta
Ibi-gun Gifu 501-0535 (JP)**

(74) Representative: **Viering, Jentschura & Partner
mbB
Patent- und Rechtsanwälte
Am Brauhaus 8
01099 Dresden (DE)**

(54) **GENE EXPRESSION PROMOTER FOR BEAUTIFUL SKIN**

(57) A gene expression promoter for skin beautification contains a fermented extract of aspergillus oryzae as an effective component. The gene expression promotor for skin beautification causes expression of at least one gene for skin beautification among BMP-2, TGFB1, CLDN7, HB-EGF, VEGF-A, LCE3D, and PPAR-D. The fermented extract of aspergillus oryzae is extracted using water after steamed rice as a raw material is fermented with aspergillus oryzae.

Figure

**Description**

TECHNICAL FIELD

The present invention relates to a gene expression promotor for skin beautification of which effective component is a fermented extract of aspergillus oryzae and that causes expression of genes for skin beautification involving beautification of human skin.

BACKGROUND ART

It was found that malted rice generates, in the process of fermentation, natural moisturizing factors such as amino acid and ceramide, and kojic acid that inhibits generation of melanin, and therefore the malted rice has been widely utilized in a moisturizing agent and a whitening agent in various products. Recently, it was revealed that koji includes a novel functional component in addition to these components. Patent Literature 1 discloses that a fermented extract of white aspergillus includes a cosmetic composition that improves a skin barrier function and a skin moisturizing function and that blocks in vivo invasion of foreign substances from outside. In order to improve the skin barrier function, this cosmetic composition promotes expression of genes CLDN1 and OCLN involving promotion of tight junction formation, promotes expression of genes TGM1 and IVL involving promotion of surface keratinization, and promotes expression of genes SPTLC2, UGCG, and GBA involving promotion of ceramide production.

CITATION LIST

Patent Literature

Patent Literature 1: Japanese Patent Application Laid-Open Publication No. 2015-027985

SUMMARY OF INVENTION

Technical Problem

Patent Literature 1 discloses that a cosmetic composition is included in a fermented extract of white aspergillus. In order to improve the skin barrier function, the cosmetic composition promotes expression of genes CLDN1 and OCLN involving promotion of tight junction formation, promotes expression of genes TGM1 and IVL involving promotion of surface keratinization, and promotes expression of genes SPTLC2, UGCG, and GBA involving promotion of ceramide production. The present applicant thought that another koji fungus might include another composition that causes expression of a gene other than the above genes that involves beautification of human skin. The present invention has is object of providing a novel gene expression promoter for skin beautification that contains a fermented extract of a koji fungus as an effective component and that promotes expression of genes contributing to beautification of human skin.

Solution to Problem

In order to achieve the above object, the present invention provides a gene expression promoter for skin beautification that contains a fermented extract of aspergillus oryzae as an effective component and that causes expression of at least one gene for skin beautification among BMP-2, TGFB1, CLDN7, HB-EGF, VEGF-A, LCE3D, and PPAR-D.

The gene expression promoter for skin beautification with the above features contains a fermented extract of aspergillus oryzae as an effective component and can cause expression of at least one gene for skin beautification among BMP-2, TGFB1, CLDN7, HB-EGF, VEGF-A, LCE3D, and PPAR-D. When expression of BMP-2 or TGFB1 is promoted by the gene expression promoter for skin beautification, the skin barrier function can be improved in a manner that differentiation of skin epidermal keratinocytes is controlled to maintain turnover in an appropriate state. When expression of CLDN7 is promoted by the gene expression promoter for skin beautification, the skin barrier function can be improved by promoting formation of tight junctions. When expression of HB-EGF is promoted by the gene expression promoter for skin beautification, a wound healing function can be improved by promoting proliferation of epidermal cells. When expression of VEGF-A is promoted by the gene expression promoter for skin beautification, the wound healing function can be improved in a manner that neovascularization is promoted by promoting proliferation of vascular endothelial cells. When expression of LCE3D is promoted by the gene expression promoter for skin beautification, a function of forming keratinized membranes of stratum epidermis can be improved in the skin epidermal keratinocytes that are epithelial cells. When expression of PPAR-D is promoted by the gene expression promoter for skin beautification, the skin barrier function can be improved by promoting differentiation of epidermal cells and lipid synthesis.

In the gene expression promoter for skin beautification with the above feature, the fermented extract of the aspergillus oryzae is preferably obtained by extraction using water after steamed rice as a raw material is fermented with the koji fungus.

BRIEF DESCRIPTION OF DRAWINGS

[FIG. 1]

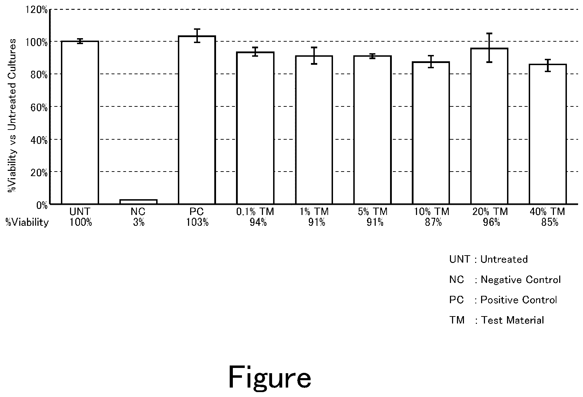

FIG. 1 is a graph representation showing results of evaluation as to cytotoxicity or non- cytotoxicity of fermented extracts from a koji fungus.

DESCRIPTION OF EMBODIMENTS

The following describes a gene expression promoter for skin beautification according to the present invention. The gene expression promoter for skin beautification according to the present invention contains a fermented extract of a koji fungus as an effective component and promotes expression of genes for skin beautification involving beautification of human skin. The fermented extract of the koji fungus contained in the gene expression promoter for skin beautification of the present invention is an extract obtained in a manner that a koji fungus fermentation product is produced by fermenting with aspergillus oryzae steamed rice, especially steamed unpolished rice, as a raw material and the produced koji fungus fermentation product is extracted using water. Note that the fermented extract of the koji fungus is not limited to an extract from unpolished rice as a raw material and may be an extract from polished rice obtained by polishing unpolished rice.

The koji fungus fermentation product is produced through the following production processes (1) and (2). The fermented extract of the koji fungus is produced by extraction through a production process (3) using the koji fungus fermentation product produced through the production processes (1) and (2).

(1) Preprocessing process of rice that is a raw material for fermentation.
(2) Koji fungus fermentation process to produce a koji fungus fermentation product by fermenting the rice with the koji fungus.
(3) Extraction process to extract a fermented extract of the koji fungus from the koji fungus fermentation product using water.

(Preprocessing Process of Rice That Is Raw Material)

In the preprocessing process of rice that is a raw material for fermentation includes washing to wash rice, immersion to immerse the washed rice in water for allowing the washed rice to absorb water, water removal to remove water attached to the surface of the rice, steaming to steam the water-absorbing rice, and inoculation to inoculate a koji fungus in the steamed rice. The washing is performed for the purpose to wash off stain and a rice bran component attached to rice that is a raw material using water. The immersion is performed for the purpose to allow the washed rice to absorb water by immersing the washed rice in water. The water removal is performed for the purpose to remove water so that water attached to the surface of the water-absorbing rice does not remain thereon. The steaming is performed for the purpose to pregelatinize starch contained in the rice subjected to immersion and water removal, and sterilize the rice. The water-absorbing rice subjected to immersion and water removal is steamed with vapor to obtain steamed rice. In the steaming, the temperature of the rice under steaming is kept at 85°C or higher for 20 to 75 minutes. Note that the temperature of the rice under steaming is preferably 90°C or higher, and more preferably 95°C or higher, and steaming time is preferably 30 to 45 minutes. The inoculation is performed for the purpose to inoculate a koji fungus in the steamed rice by spraying spores of the koji fungus in a powder state after the steamed rice is cooled to 37°C or lower. The water content of the steamed rice is preferably 20 to 50%, which is suitable for inoculation, and more preferably 30 to 40%. In order to spray the spores of the koji fungus evenly on the surface of the steamed rice, seed malt may be used that is obtained by mixing an excipient such as starch or rice powder with the spores of the koji fungus in a powder state.

(Koji Fungus Fermentation Process)

The koji fungus fermentation process is performed for the purpose to produce a koji fungus fermentation product from the steamed rice with the koji fungus by propagation of the inoculated koji fungus on the steamed rice. As to the temperature suitable for the koji fungus fermentation process, the temperature of the steamed rice is kept at 20 to 45°C, and preferably kept at 30 to 40°C. The concentration of carbon dioxide suitable for the fermentation process is kept at no greater than 10%, and preferably kept at no greater than 5%. The fermentation process is preferably performed under a high-humidity condition. As to the humidity suitable for the fermentation process, the humidity is kept at 40 to 100%, preferably kept at 60 to 100%, and further preferably at 80 to 100%. Preferably, koji is allowed to grow in the fermentation time until the hyphae of the koji fungus covers the entire surface of the steamed rice. Specifically, the fermentation time is such that the koji is allowed to grow for 40 to 120 hours, more preferably 80 to 120 hours under fermentation conditions of for example the fermentation temperature, the carbon dioxide concentration, and the fermentation humidity described above.

In the koji fungus fermentation process, drying may be performed in order to make preservability excellent until the extraction process is performed, by reducing the content of water contained in the koji after the fermentation process. In the drying, moisture contained in the koji is evaporated by blowing hot air to the koji after the fermentation. The temperature of the hot air blown to the koji is preferably 25 to 50°C, and more preferably 30 to 45°C. The koji is dried with the hot air until the content of water contained in the koji after the drying is no greater than 10%, and more preferably no greater than 5%. Note that in a case in which the extraction process is performed directly after the fermentation process, the drying may be omitted.

(Extraction Process)

The extraction process is performed for the purpose to extract a fermentation product included in the koji. In the present embodiment, various components included in the koji fungus fermentation product produced in process of the koji fungus fermenting the rice are extracted using water for extraction of a fermented extract of a koji fungus.

The fermented extract of the koji fungus produced through the production processes (1) to (3) contains a gene expression promoter for skin beautification that causes expression of genes for skin beautification involving beautification of human skin, and accordingly can be taken to be a gene expression promoter for skin beautification containing the fermented extract of the koji fungus as an effective component. Here, examples of genes for skin beautification expressed by the gene expression promoter for skin beautification contained in the fermented extract of the koji fungus include BMP-2, TGFB1, CLDN7, HB-EGF, VEGF-A, LCE3D, and PPAR-D, and is only required to include at least one (one or more) of BMP-2. TGFB1, CLDN7, HB-EGF, VEGF-A, LCE3D, and PPAR-D.

When expression of BMP-2 or TGFB1 is promoted by the gene expression promoter for skin beautification contained in the fermented extract of the koji fungus, the skin state appropriate for turnover can be maintained by controlling differentiation of skin epidermal keratinocytes to improve the skin barrier function. When expression of CLDN7 is promoted by the gene expression promoter for skin beautification contained in the fermented extract of the koji fungus, tight junction formation can be promoted to improve the skin barrier function. When expression of HB-EGF is promoted by the gene expression promoter for skin beautification contained in the fermented extract of the koji fungus, proliferation of epidermal cells can be promoted to improve a wound healing function.

When expression of VEGF-A is promoted by the gene expression promoter for skin beautification contained in the fermented extract of the koji fungus, neovascularization can be promoted by proliferation of vascular endothelial cells to improve the wound healing function. When expression of LCE3D is promoted by the gene expression promoter for skin beautification contained in the fermented extract of the koji fungus, a function of forming keratinized membranes of stratum epidermis can be improved in skin epidermal keratinocytes that are epithelial cells. When expression of PPAR-D is promoted by the gene expression promoter for skin beautification contained in the fermented extract of the koji fungus, differentiation of epidermal cells and lipid synthesis can be promoted to improve the skin barrier function.

The fermented extract of the koji fungus contains the gene expression promoter for skin beautification that causes expression of the genes for skin beautification involving beautification of human skin. A cosmetic material composition can be produced by mixing at least one of a surfactant, gold colloid, a glucosamine derivative, and a vitamin B derivative with a cosmetic composition with the gene expression promotor for skin beautification contained in the fermented extract of the koji fungus as an effective component. Specifically, the cosmetic material composition can serve as a serum, a makeup cream, a lotion, a body gel, or an emulsion, for example.

Examples

The following specifically describes an example of the present invention.

(Preprocessing Process of Rice That Is Raw Material)

In the preprocessing process of rice that is a raw material, 10 kg of rice that is the raw material is washed with water first in the washing to wash off stain and a rice bran component attached to the surface of the rice. Next, the washed rice is immersed in water for 6 hours in water absorption and immersion, and the immersed rice is then subjected to water removal using a strainer such as a sieve for 12 hours. Next, the rice subjected to the water removal is steamed for 45 minutes using a steamer while the product temperature (temperature of the rice) is kept at 95°C or higher, and the resultant rice is taken to be steamed rice. Finally, after the steamed rice is allowed to cool to 40°C, spores of aspergillus oryzae that is a koji fungus are inoculated in the cooled steamed rice in the inoculation. The spores of the koji fungus in a powder state is evenly sprayed on the surface of the steamed rice using a sprayer, and the resultant steamed rice is mixed so that the spores of the koji fungus are evenly distributed throughout the steamed rice.

(Koji Fungus Fermentation Process)

In the koji fungus fermentation process, the koji is produced using an automatic koji making apparatus for appropriate temperature management. The temperature was controlled so that the product temperature was 37°C for 25 hours from the start of fermentation and the product temperature was 35°C after 25 hours from the start of fermentation. Furthermore, the humidity is controlled so that the humidity in the automatic koji making apparatus is 60 to 100% all the time during fermentation, and the concentration of carbon dioxide in the automatic koji making apparatus is controlled to be no greater than 10%. Note that the koji fungus fermentation process ends once the entire surface of the steamed rice is covered with the hyphae of the koji fungus. In a case in which the fermented extract of the koji fungus is not immediately extracted from malted rice (a koji fungus fermentation product) produced in the koji fungus fermentation process, the malted rice is dried by hot-air blowing so as to reduce the content of water.

(Extraction Process)

A water-soluble component contained in the malted rice is extracted using water by adding 400 ml of purified water to 100 g of the malted rice that is the koji fungus fermentation product and continuously stirring the resultant mixture for 3 hours. An aqueous extract is coarsely filtered using a filter (product of ADVANTEC TOYO KAISHA, LTD., quantitative filter No. 2, circle), and the resultant filtrate is then finely filtered using a membrane filter (Millex-HV filter, product of MERCK, maximum pore diameter 0.45 μm, hydrophilic polyvinylidene fluoride (PVDF)) to collect an aqueous fermented extract of the koji fungus containing a fermented extract of the koji fungus.

(Method of Cytotoxicity Test on Aqueous Fermented Extract of Koji Fungus)

Whether or not the fermented extract of the koji fungus has cytotoxicity is determined by MTT assay using a three-dimensional culture model of human skin epidermis. First, the aqueous fermented extract of koji fungus is adjusted to have a concentration of 0.1%, 1%, 5%, 10%, 20%, or 40% using Dulbecco's phosphate buffered saline (DPBS). Then, 25 μL of the aqueous fermented extract of the koji fungus adjusted at any of the concentrations is applied onto the surface of cells. Note that the condition for negative control (NC) is that 100 μL of Triton X-100 at a concentration of 1% is applied while the condition for positive control (PC) is that any samples are not at all applied onto the surface of the cells. The cells to which the test sample has been applied are cultivated for 24 hours using a carbon dioxide gas incubator under conditions of a concentration of $CO_2$ of 5% and a temperature of 37°C. After the 24 hours, the test sample attached to the surface of each cell is washed off using DPBS. The cultivated cells subjected to the above treatment are moved to a 24-hole plate, and 300 μL of a MTT culture medium containing 1 mg/ml of a MTT powder was added thereto. Then, the resultant cells are left to stand for 3 hours using a carbon dioxide gas incubator under conditions of a concentration of $CO_2$ of 5% and a temperature of 37°C. The cultivated cells in each hole are roughly washed with a DPBS solution and moved to holes of another 24-hole plate. Into each hole of the 24-hole plate into which the cultivated cells have been moved, 2 ml of isopropyl alcohol at a concentration of 100% is added and extraction is performed for 2 hours at room temperature under shaking. Each hole of a 96-hole plate is filled with 200 μL of the resultant aqueous extract, and the absorbance of the aqueous extract with respect to light with a wavelength of 570 nm is measured using a microplate reader.

(Result of Cytotoxicity Test on Aqueous Fermented Extract of Koji Fungus)

A viability is calculated using the following calculation formula.

$$\text{Viability rate }(\%) = (\text{A570 at processing on each sample group/A570 before processing}) \times 100$$

FIG. 1 shows the cell viabilities in treatment with the aqueous extracts of the fermented koji fungus at respective concentrations. As shown in graphs for 0.1% TM to 40% TM in FIG. 1, the cell viabilities were 85 to 94%, and no significant difference was recognized from a result of a significant difference test. From the above, it is revealed that the fermented extract of the koji fungus contained in the aqueous fermented extract of the koji fungus has no cytotoxicity, that is, promotion of expression of genes for skin beautification by the aqueous fermented extract of the koji fungus is not caused by cell death.

(Evaluation of Promotion of Expression of Genes for Skin Beautification by Aqueous Fermented Extract of Koji Fungus)

Onto the surface of a three-dimensional culture model of human skin epidermis set on a 24-hole plate, 25 µL of a solution of the aqueous fermented extract of the koji fungus at a concentration of 1% or 25 µL of a solution of the aqueous fermented extract of the koji fungus at a concentration of 40% is applied. Here, each of the solutions is diluted with Dulbecco's phosphate buffered saline (DPBS). Cultivation is performed for 24 hours using a carbon dioxide gas incubator under conditions of a concentration of $CO_2$ of 5% and a temperature of 37°C. The cultivated cells in each hole are roughly washed with a DPBS solution and then moved into holes of another 24-hole plate. RNA, DNA, and protein in the collected cells are stabilized. RNAlater that is an aqueous solution for protection from decomposition is then added. After incubation is performed at 4°C for 4 days, mRNA is extracted from each tissue. The extracted mRNA is synthesized into cDNA by the RT-PCR method using a reverse transcriptase high capacity cDNA synthesis kit. The resultant cDNA is molded into a mold, and real-time PCR is performed to quantitate each DNA amount of BMP-2, TGFB1, CLDN7, HB-EGF, VEGF-A, LCE3D, and PPAR-D.

As shown in Table 1, when the solution of the aqueous fermented extract of the koji fungus at a concentration of 1% was added to the cell culture liquid, expression of BMP-2 was promoted by 130%, expression of VEGF-A was promoted by 30%, expression of LCE3D was promoted by 49%, and expression of TGFB1 was promoted by 20%. Also, when the solution of the aqueous fermented extract of the koji fungus at a concentration of 40% was added to the cell culture liquid, expression of BMP-2 was promoted by 270%, expression of TGFB 1 was promoted by 29%, expression of CLDN7 was promoted by 78%, expression of HB-EGF was promoted by 93%, expression of VEGF-A was promoted by 78%, expression of LCE3D was promoted by 106%, and expression of PPAR-D was promoted by 53%. From the above, it was revealed that the fermented extract of the koji fungus contained in the aqueous fermented extract of the koji fungus contains a gene expression promotor for skin beautification involving beautification of human skin. Use of a cosmetic composition with the fermented extract of the koji fungus as an effective component to human skin can contributed to beautification of the human skin safely at low cost.

[Table 1]

| Gene for skin beautification | 1%-Aqueous fermented extract of koji fungus | 40%- Aqueous fermented extract of koji fungus |
|---|---|---|
| BMP-2 | +130% | +270% |
| CLDN7 | n.s. | +78% |
| LCE3D | +49% | +106% |
| PPAR-D | n.s. | +53% |
| VEGF-A | +30% | +78% |
| HB-EGF | n.s. | +93% |
| TGFB1 | +20% | +29% |

**Claims**

1. A gene expression promoter for skin beautification comprising a fermented extract of aspergillus oryzae as an effective component, wherein
   the gene expression promotor for skin beautification causes expression of at least one gene for skin beautification among BMP-2, TGFB1, CLDN7, HB-EGF, VEGF-A, LCE3D, and PPAR-D.

2. The gene expression promoter for skin beautification according to claim 1, wherein
   the fermented extract is an extract from steamed rice as a raw material fermented with the aspergillus oryzae, the extract being extracted into water.

Figure

UNT : Untreated
NC  : Negative Control
PC  : Positive Control
TM  : Test Material

## INTERNATIONAL SEARCH REPORT

International application No.

PCT/JP2020/015809

### A. CLASSIFICATION OF SUBJECT MATTER

Int. Cl. A61P43/00(2006.01)i, A61P17/00(2006.01)i, A61Q19/00(2006.01)i, A61K36/062(2006.01)i, A61K36/899(2006.01)i, A61K8/9728(2017.01)i, A61K8/9794(2017.01)i
FI: A61K8/9728, A61P17/00, A61P43/00 111, A61K36/062, A61P43/00 107, A61K36/899, A61P43/00 121, A61Q19/00, A61K8/9794

According to International Patent Classification (IPC) or to both national classification and IPC

### B. FIELDS SEARCHED

Minimum documentation searched (classification system followed by classification symbols)
Int. Cl. A61P43/00, A61P17/00, A61Q19/00, A61K36/062, A61K36/899, A61K8/9728, A61K8/9794

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2017-071578 A (FUKUOKA-KEN) 13 April 2017 | 1-2 |
| A | JP 2001-089314 A (GOLD ONE KK) 03 April 2001 | 1-2 |

☐ Further documents are listed in the continuation of Box C.   ☒ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 04.06.2020 | 16.06.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/015809

| Patent Documents referred to in the Report | Publication Date | Patent Family | Publication Date |
|---|---|---|---|
| JP 2017-071578 A | 13.04.2017 | (Family: none) | |
| JP 2001-089314 A | 03.04.2001 | (Family: none) | |

Form PCT/ISA/210 (patent family annex) (January 2015)

**EP 3 967 370 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015027985 A